# EUROPEAN PATENT APPLICATION

(11) **EP 3 964 759 A1**
(43) Date of publication of application: **09.03.2022**
(21) Application number: 20382789.4
(22) Date of filing: 04.09.2020
(51) Int. Cl.: F24F 8/15, F24F 8/22, F24F 8/26, F24F 8/30, F24F 11/58, F24F 110/10, F24F 110/20, F24F 110/66, F24F 110/70, F24F 110/72, A61L 9/20, A61L 9/22

(54) **DEVICE FOR GENERATING HYDROXYL RADICALS**

(71) Applicant: Novoa Diz, Susana, 08750 Molins de Rei Barcelona (ES)
(72) Inventor: BECERRIL RUIZ, JOSE LUIS, 08750 MOLINS DE REI (BARCELONA) (ES)
(74) Representative: Durán-Corretjer, S.L.P.

(57) **Abstract**

This invention refers to a device for generating hydroxyl radicals comprising: a) at least one container suitable for containing a substance from which hydroxyl radicals are generated; b) at least one conversion unit suitable for generating hydroxyl radicals from that substance; and c) a base comprising a power supply, a fan, an air inlet and outlet duct from the device, means for electrically supplying the various components of the device and a controller; in which the air inlet and air outlet duct include a mixing vessel before the air outlet of the device, characterised in that these at least one vessel and at least one conversion unit are independent of each other and can be removed from the base unit independently of each other.

## Description

The present invention relates to a device for the sterilisation of indoor environments through the generation of hydroxyl radicals (•OH), ions and other radicals. With the device of the present invention it is possible to very effectively eliminate pollutants of biological origin such as bacteria and viruses, as well as pollutants present in the air, such as carbon monoxide, sulphur dioxide or nitrogen dioxide.

With the development of industrialisation and the increase of the world population, air pollution has increased and every day new pathologies related to agents of biological origin, such as viruses and bacteria appear, which makes pollution in closed spaces more serious. In addition, the various sources of resources used to produce the energy required for human activity continue to release pollutants into the air that are harmful to human and animal health. On the other hand, the increase in population density favours contagion between individuals, increasing the risk in indoor environments, which makes it necessary to establish distance measures and continuous cleaning and sterilisation processes for surfaces exposed to possible contamination. All these factors increase the need for continuous treatment of air and surfaces that may have a contaminating load.

Contaminated air can contain pathogens such as bacteria, viruses and fungi, which often live in humid environments. The inhalation of bacterial or fungal spores or viral particles in suspension is the cause of a wide range of diseases. The air inhaled by people, contaminated with these microorganisms, and which may also contain volatile organic compounds, can be the cause of several diseases and can lead to systemic infections and allergies.

Nowadays, many technologies using different methods for the removal or adsorption of these pollutants exist. One example is the use of filtration means, where all the air has to be sucked through the filter, which must then be cleaned or replaced every time the saturation level is reached. The efficiency of a filtration system is also limited by the size of the contaminating particles, as filters are not adapted to particles of very different sizes. Filters also trap biological matter, which is a point of contagion if replacement or maintenance is not carried out correctly. In addition, filter systems have no effect on surfaces, their range of action is limited and they have a high cost.

On the other hand, there are ion generators that increase the ion charge in the air by generating and releasing mainly negatively-charged ions into the air. One application would be, for example, PCl technology (Plasma Cluster lonisation), which consists in the generation of plasma ions, releasing positive and negative ions. The charged particles then adhere to surfaces in the room, where they can cause allergies or asthma problems. In addition, they can produce dangerous levels of ozone and do not produce hydroxyl radicals.

Photocatalysis oxidation (PCO) technology consists of irradiation with ultraviolet light of surfaces coated with titanium dioxide (TiO₂) to generate free radicals. With this, all the air has to be passed through the device. Most studies concur it is effective only on surfaces coated with TiO₂, which means that it only acts on pollutants that are in contact with these surfaces or very close to them, and therefore its effectiveness is limited. Although some of said devices can release hydroxyl radicals, tests have concluded that they produce very low quantities with a high dependence on relative ambient humidity.

In conclusion, most state-of-the-art systems are deficient, either because they are only effective for specific pollutants, because they treat only part of the indoor air but not the surfaces, because they generate by-products that can be harmful to health in general, or because they cannot be in constant operation or used when people are present. In addition, they all have high energy consumption. It is therefore necessary to develop a device that can overcome the abovementioned shortcomings.

The inventors of the present invention have developed a device for generating hydroxyl radicals (•OH) which improves hygiene conditions in indoor spaces by acting on the air and on soft and porous solid surfaces, such as fabrics, present in those spaces and thus improving the quality of life of humans in particular, and other living beings in general, with very low energy consumption.

The device, for generating hydroxyl radicals of the present invention also allows the generation of other radicals, such as the hydroperoxyl radical (•HO₂), and of negative and/or positive ions.

The radicals •OH and •HO₂ are formed naturally when ultraviolet sunlight reacts with water vapour (H₂O) and ozone (O₃) in the lower layers of the atmosphere. The hydroxyl radical (•OH), a molecule formed by a hydrogen atom and an oxygen atom with an unpaired electron, is one of the most reactive gases in the atmosphere. It acts as a "detergent" in the air, breaking down other gases and killing viruses and bacteria. In particular, •OH is the main controller on methane concentrations , a powerful greenhouse gas, that is the second largest contributor to global warming after carbon dioxide.

•OH plays a very important role in the natural purification of pollutants as it is the main cleaning agent of the atmosphere, removing several pollutants such as carbon monoxide, sulphur dioxide and nitrogen dioxide. Hydroxyl radicals are present in nature with a density of 2 to 20 million per cubic centimetre of air. •OH radicals have been shown not only to be completely harmless to the health of plants, animals and humans, but also to act effectively in the air, on surfaces of solid materials, as well as in textiles and other porous materials.

Therefore, this present invention discloses a device for generating hydroxyl radicals comprising:
a) at least one container suitable for containing a substance from which said hydroxyl radicals are generated;
b) at least one conversion unit, suitable for generating said hydroxyl radicals from said substance; and
c) a base comprising a power supply, a fan, an air inlet and outlet duct of the device, means for electrically supplying the various components of the device and a controller; wherein the air inlet and outlet duct of the device comprises a mixing vessel before the air outlet of the device,
characterised in that said at least one container and at least one conversion unit are independent of each other and can be removed from the base unit.

The device of the present invention has the advantage that both the conversion unit and the container of the substance from which hydroxyl radicals are generated are independent of each other, i.e. they are not part of the same cartridge, and moreover they are removable, so that each one can be easily replaced, for example manually, retaining only the base. This allows for a reduction of the cost of the device, since it is not necessary to replace the container and the conversion unit at the same time. This advantage with respect to state-of-the-art devices is especially important since the substance from which hydroxyl radicals are generated is exhausted long before the conversion unit, so the device of the present invention has the advantage that the substance container can be replaced without having to replace the conversion unit at the same time.

In addition, the fact that both the conversion unit and the container can be disassembled and replaced independently of each other allows multiple combinations of the substance and the conversion unit to suit different applications of the device. Thus, the generation of hydroxyl radicals, ions and other radicals can be optimised according to various parameters, such as the charge or quantity required of each of them depending on the different types of contaminants or the location or size of the application. Therefore, the device of the present invention allows total adaptability to the specific conditions of each application.

As already detailed above, the device for generating hydroxyl radicals of the present invention has a base comprising an air inlet and outlet duct from the device.

Preferably, said air inlet and outlet duct comprises a first section, which conducts the air supplied by the fan; a second section connected to the first, which is narrower and to which the substance generated from the container arrives; and a third section connected to the second and first, which conducts the mixture of the fluids coming from the first two sections to the outlet of the device. Preferably, the third section comprises the mixing vessel of the air inlet and the outlet duct. It is in this mixing vessel that the reaction that generates the hydroxyl radicals is completed before they are released from the device.

In a preferred embodiment, the first section of the air inlet and outlet duct of the device comprises a primary air inlet channel, while the second section of the air inlet and outlet duct of the device comprises a secondary air inlet channel. Said primary air inlet channel is preferably located in the area of the fan and the secondary air inlet channel is preferably located in the area of the container and the conversion unit.

More preferably, the first and second section of the air inlet and outlet duct have respective diameters to create a Venturi effect to attract the air from the first and second section into the duct. Similarly, the third section has a diameter that will create a Venturi effect to draw the air coming from the first and second sections. The person skilled in the art knows the diameter each section of the air duct should have for the Venturi effect to be created. A Venturi effect is a phenomenon by which the pressure of a moving fluid within a closed duct is decreased when its speed increases as it passes through an area of smaller section. When said speed increases very much, large differences in pressure can occur and then, if at that duct end another duct is introduced, then the fluid from that duct will be sucked in and mixed with the fluid circulating along the first duct.

Preferably, the container, also called the flask, is an open container containing a wick of a material selected from the list comprising cellulose, cotton, sponge or a mixture of these. Said wick has the function of assisting in the evaporation of the substance or reagent from which the hydroxyl radicals are generated, so that it can be more easily transferred to the mixing vessel. Preferably, the substance from which hydroxyl radicals are generated will be a liquid selected from the group comprising terpenes such as limonene, myrcene, pinene, linalool or rosemary essential oil, hydrogen peroxide, water or a mixture of these. Preferably, said container does not include any electrical connections.

Preferably, the conversion unit is be a plasma generator, capable of generating ions and small amounts of ozone, which react with the substance from which these hydroxyl radicals are generated, oxidising it and generating large amounts of them.

In another embodiment, said conversion unit can be a generator of ultraviolet light with a wavelength between 200 nm and 300 nm, generated by either lamps or light-emitting diodes (LEDs), which through photolysis are able to react with the substance from which hydroxyl radicals are generated without the use of any catalytic compound such as titanium dioxide (TiO₂) and without generating ozone.

Optionally, the device of the present invention may include a high frequency ultrasonic generator. By generating high frequency ultrasounds, usually in the range of 1.5 MHz to 2 MHz, the substance from which hydroxyl radicals are generated is dissociated. This method can be used both in air by acting on the gaseous phase of the substance from which hydroxyl radicals are generated, and by direct irradiation of the substance from which hydroxyl radicals are generated, in its liquid form. This produces cavitation bubbles that repeatedly grow and collapse. During the collapse, temperature and pressure increase exponentially causing the generation of large amounts of free radicals. In this case no ozone is generated. More preferably, the ultrasound generator may be installed in the mixing vessel or in the container. The ultrasound generator may be installed so that it only irradiates air with molecules of the substance from which hydroxyl radicals are generated, but can be installed immersed in the substance.

In a preferred embodiment, the conversion unit of the hydroxyl radical generator of this invention is an ozone (O₃) generator.

In a preferred embodiment, the conversion unit of the hydroxyl radical generator of this invention is a generator of positive and/or negative ions.

In the device from this invention, it is contemplated that more than one conversion unit may be used. It is also contemplated that when the conversion unit is an ultraviolet light generator, said conversion unit is installed in the container.

Preferably, the device from this invention also comprises sensors for the detection of volatile organic compounds, or sensors for particles in the air, or sensors for temperature and humidity, or sensors for the detection of carbon monoxide or carbon dioxide, among other. The purpose of these sensors is to determine the air quality.

Preferably, the device from this invention further comprises a data transmission module, for example, by means of Wi-Fi and/or Bluetooth, that allows an exchange of information with other devices. In this way, it is possible to control air quality remotely, as well as to control the device by means of mobile phones, tablets or any other device with Wi-Fi and/or Bluetooth connectivity.

Some embodiments of this invention are further detailed below with reference to the attached schematic figures in which:
Figure 1 shows a schematic view of an embodiment of the base and the conversion unit of the hydroxyl radical generating device according this invention.
Figure 2 shows a schematic view of an embodiment of the base, the conversion unit and the container of the substance from which hydroxyl radicals are generated according this invention.
Figure 3 shows a schematic view of an embodiment of the device according this invention in which the way in which the conversion unit and the substance container are attached to the base of said device is shown.
Figure 4 shows a schematic view of an embodiment of the device according this invention in which the conversion unit and the container of the substance have been placed at the base of the device.
Figure 5 shows a schematic view of an embodiment of the device according this invention in which the way in which the air circulates in the device is shown.
Figure 6 shows a schematic view of another possible embodiment of the device according this invention.

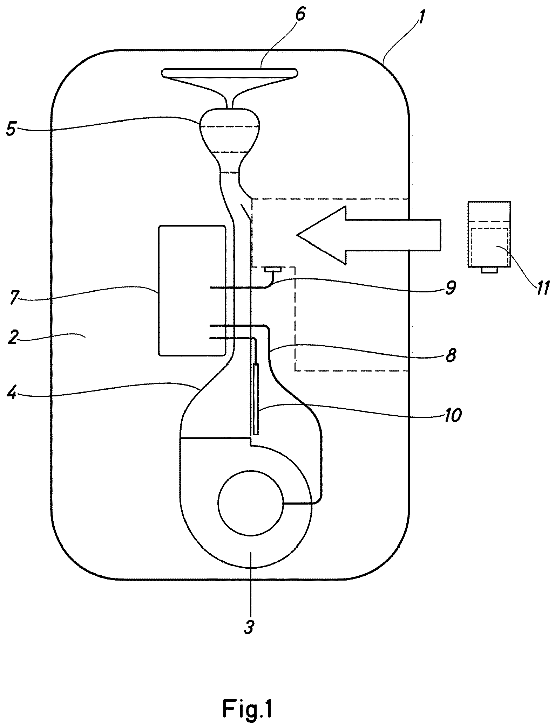

As shown in Figure 1, the device 1 from this invention comprises a base 2, in which there is a fan 3, an air duct 4 comprising a mixing vessel 5 near the air outlet 6 of the device. In addition, said base 2 comprises a controller 7 in the form of a base plate, a sensor 10, and means 8, 9 for electrical connections to the various components of the device. The arrow shows the position in which the conversion unit 11, which in this case is an ionizer, is placed in the base 2. The air duct 4 has a wider first section and a narrower second section that gets even more narrower before the connection to the secondary channel.

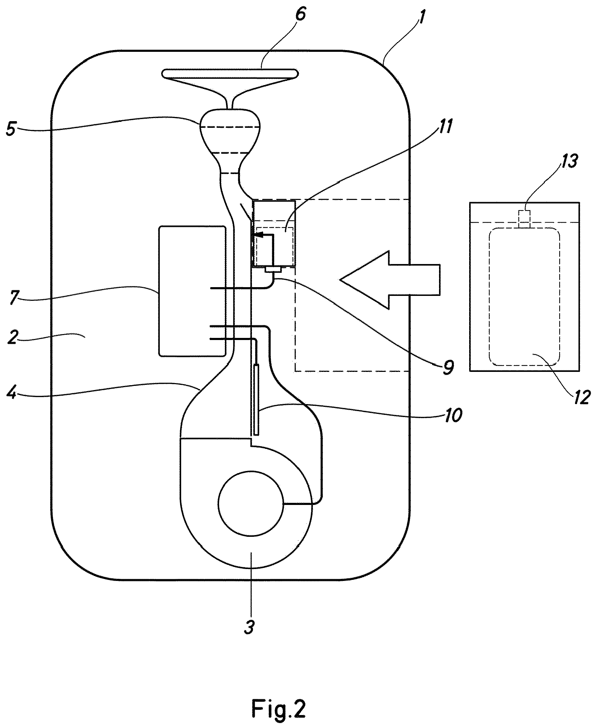

Figure 2 shows the conversion unit (ioniser) 11 placed in its position and shows a vessel 12 suitable to contain a substance from which hydroxyl radicals can be generated. The arrow indicates the position of container 12 in base 2. Said container includes a wick 13, to assist in the evaporation of the substance or reagent present in said container 12.

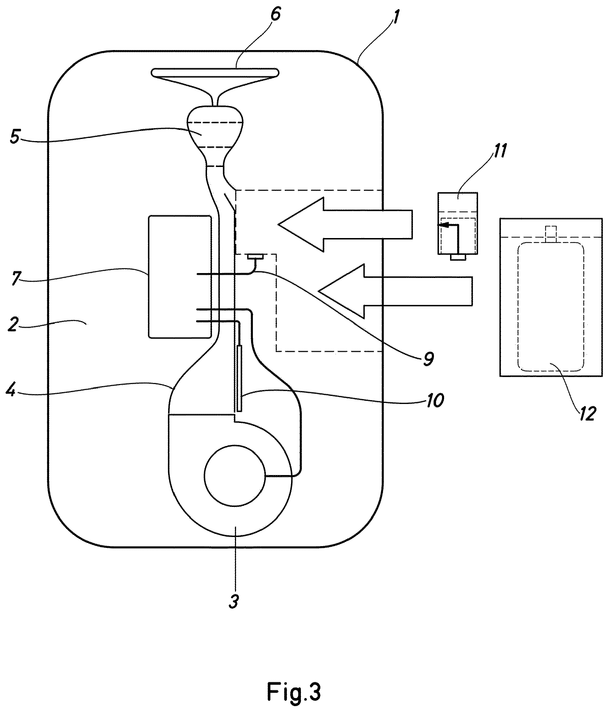

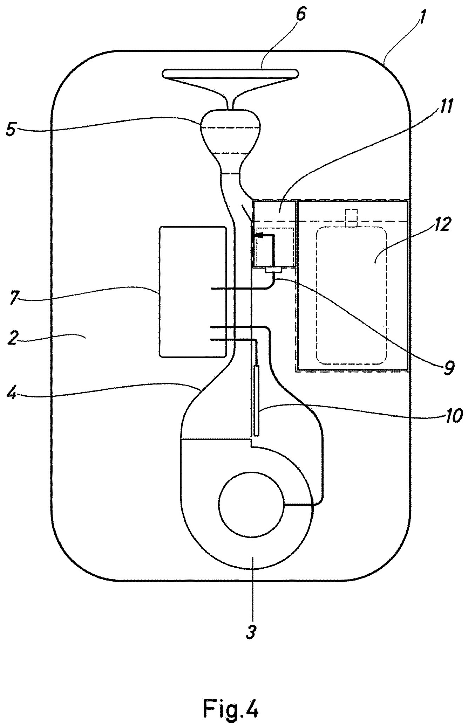

Figures 3 and 4 show how the conversion unit 11 and container 12 are placed in the base 2. The arrows (Figure 3) indicate how both should be placed, independently of each other. The container or cartridge containing the reagent does not have any electrical connections, however, the conversion unit 11, whether in the form of an ioniser or plasma generator or other, has an electrical connection 9.

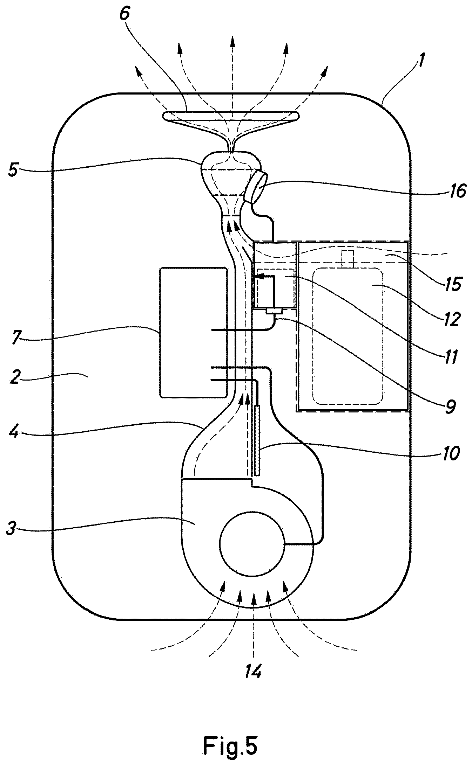

As shown in Figure 5, outside air enters through the primary channel 14, is absorbed by the fan 3 and led to the air inlet and outlet duct 4 through the first and second section. This arrangement gives the air an acceleration and a pressure increase that causes a Venturi effect, allowing the total absorption of the air in the secondary channel 15 that contains the hydroxyl radicals generated by the conversion unit and the substance or reagent. Thus, both the fluid coming from the primary channel 14 and the one absorbed from the secondary channel 15 are mixed and go into the mixing vessel 5 where a turbulence that encourages the process of hydroxyl radical generation is produced. The air with the hydroxyl radical load is then expelled to the outside through the air outlet 6.

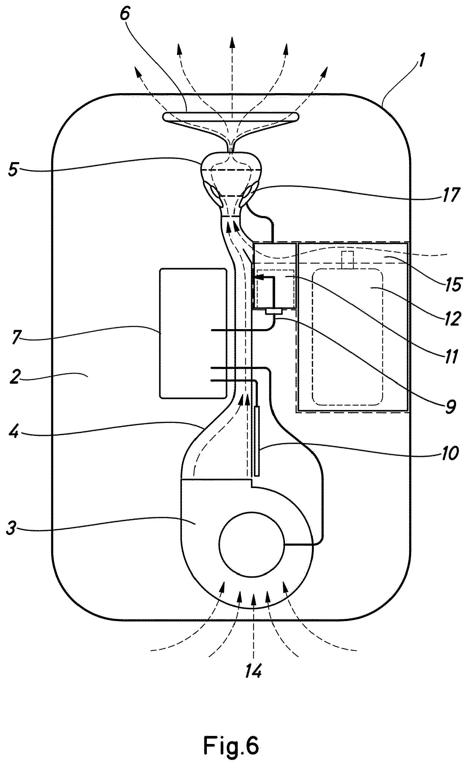

Optionally, the mixing vessel 5 can have a coating of parallel lines or in the form of a mesh made from a conductive metal material (not shown) and an ultrasonic generator 16 (Figure 5) or ultraviolet light 17 (Figure 6). Said generator improves the excitation and/or vibration of hydroxyl radical generator molecules, an effect that is enhanced by the metal cladding of the mixing vessel 5 and that allows for better mixing.

In order to cover a large area of interior space, the hydroxyl radical generator is able to generate a stable and sufficient amount of hydroxyl radicals through various reactions and exhibit superior effects in purifying and sterilising the air, therefore providing a higher quality of life and environment than is provided by currently available devices.

## Claims

1. A device for generating hydroxyl radicals comprising:
a) at least one container suitable for containing a substance from which said hydroxyl radicals are generated;
b) at least one conversion unit, suitable for generating said hydroxyl radicals from said substance; and
c) a base comprising a power supply, a fan, an air inlet and outlet duct of the device, means for electrically supplying the various components of the device and a controller; wherein the air inlet and outlet duct of the device comprises a mixing vessel before the air outlet of the device,
**characterised in that** said at least one container and at least one conversion unit are independent of each other and can be removed from the base unit.

2. A device for generating hydroxyl radicals, according to claim 1, **characterised in that** the air inlet and outlet duct of the device comprises a first section, which conducts the air supplied by the fan; a second section connected to the first, which is narrower and where the substance generated from the container arrives; and a third section connected to the second and first, which conducts the mixture of the fluids from the first two sections to the device outlet.

3. A device for generating hydroxyl radicals, according to claim 2, **characterised in that** the first section comprises a primary air inlet channel, and the second section comprises a secondary air inlet channel.

4. A device for generating hydroxyl radicals, according to any one of the preceding claims, **characterised in that** the container is an open container comprising a wick of a material selected from the list comprising cellulose, cotton, sponge or a mixture thereof.

5. A device for generating hydroxyl radicals, according to any one of the preceding claims, **characterised in that** the substance from which hydroxyl radicals are generated is a liquid substance which is selected from the group comprising terpenes such as limonene, myrcene, pinene, linalool or rosemary essential oil, hydrogen peroxide, water or a mixture thereof.

6. A device for generating hydroxyl radicals, according to any one of the preceding claims, **characterised in that** the device also generates other radicals, such as hydroperoxyl radicals (-HO₂) and/or negative and/or positive ions and/or ozone (O₃).

7. A device for generating hydroxyl radicals, according to any one of the preceding claims, **characterised in that** its conversion unit is a plasma generator.

8. A device for generating hydroxyl radicals, according to any one of claims 1 to 6, **characterised in that** its conversion unit is an ultraviolet light generator.

9. A device for generating hydroxyl radicals, according to claim 8, **characterised in that** said ultraviolet light generator generates ultraviolet light with a wavelength between 200 nm and 300 nm.

10. A device for generating hydroxyl radicals, according to any one of claims 1 to 6, **characterised in that** said conversion unit is an ultrasound generator.

11. A device for generating hydroxyl radicals, according to claim 10, **characterised in that** said ultrasound generator generates at a frequency in the range of 1.5 MHz to 2 MHz.

12. A device for generating hydroxyl radicals, according to any one of claims 1 to 6, **characterised in that** said conversion unit is an ion generator.

13. A device for generating hydroxyl radicals, according to any one of claims 1 to 6, **characterised in that** said conversion unit is an ozone (O₃) generator.

14. A device for generating hydroxyl radicals, according to any one of the preceding claims, **characterised in that** it also comprises sensors for the detection of volatile organic compounds, or sensors for particles in the air, or temperature and humidity sensors, or sensors for the detection of carbon monoxide or carbon dioxide.

15. A device for generating hydroxyl radicals, according to any one of the preceding claims, **characterised in that** it also comprises a data transmission module for either Wi-Fi and/or Bluetooth transmissions.
